# EUROPEAN PATENT SPECIFICATION

(11) **EP 0 596 161 B1**
(45) Date of publication and mention of the grant of the patent: **04.02.1998**
(21) Application number: 92203427.7
(22) Date of filing: 06.11.1992
(51) Int. Cl.: A61M 5/42, A61M 37/00

(54) **Apparatus for subcutaneous introduction of a needle**
Vorrichtung zum subkutanen Einführen einer Nadel
Dispositif pour l'introduction souscutanée d'une aiguille

(43) Date of publication of application: 11.05.1994
(73) Proprietor: TEXAS INSTRUMENTS INCORPORATED, Dallas Texas 75265 (US); Texas Instruments Holland B.V., 7600 AA Almelo (NL)
(72) Inventor: Van Der Aa, Bartholomeus Wilhelmus Johannes, NL-2611 LB Delft (NL)
(74) Representative: de Bruijn, Leendert C.

(56) References cited:
- DE-B- 1 060 099
- GB-A- 2 138 298
- NL-A- 8 901 858
- US-A- 3 324 854

## Description

The invention relates to an apparatus according to the preamble of claim 1. Such an apparatus is known from US-A-3,324,854. In that specification a syringe needle is described for intravaneous introduction. Resilient guiding members are provided connected to each other at their free extremity which function to keep the part of the skin clamped between them to promote introduction of the needle. The needle is displaceable with regard to the guiding members.

The invention aims to provide an apparatus for subcutaneous introducing of articles such as transponders. Such an apparatus is known from NL-A-9100160 (Texas Instruments Holland B.V.)

In that application the use of a guiding rod is described extending in the same direction as the needle. This guiding rod is spaced from the needle with a distance corresponding to the thickness of the skin. In the position of use the guiding rod is above the needle and the skin is in the clearance between the needle and the guiding rod. Such an apparatus is especially realized for introduction in parts of an animal which are extremely rounded. As example the head of an animal is shown and the needle is introduced in the transfer face between the horizontal and vertical parts of the head near the ear.

However, such a device will not function if the needle has to be introduced in a relatively flat portion of a living being. Needles are inserted for introduction of all kinds of fluids, such as drugs, as well as solid products. The solid products can comprise drugs and transponders for identifying the related animal. Often it is important that there is a guarantee that the needle slides between the skin and the bone cartilage or tissue being immediately under the skin. On the one hand damage to the bone has to be prevented and on the other hand exiting of the needle through the skin with its tip will result in a failure of introduction of whatever product which has to be introduced.

The invention aims to overcome this problem and to provide an apparatus as above which is suitable to introduce a needle below the skin in a relatively flat portion of an animal.

According to the invention this is realized with the characterizing features of the main claim.

Through the use of rigid guiding members which are fixed relative to the needle positive introduction of the needle is provided. To be able to introduce the needle its extremity has to extend beyond the curved end portion of the guiding members.

Through the use of two spaced guiding members between which the needle is positioned optimum guidance of the needle during introduction can be guaranteed. The externally acting lower surface of the guiding members should be at least at the same level of the lower surface of the needle which has to be introduced. This means that depending from the thickness of the skin of the related animal its skin is somewhat curved around the needle. In this way loosening of the skin from the tissue below is promoted. If the skin of the animal is not very thick or introduction of the related product has to be nearer to the skin of the animal the lower face of the guiding members contacting the skin of the animal has to be at least level top surface of the needle, i.e. the curvature of the skin between the guiding members and the needle is more considerable. Of course it is possible to further rise the position of the needle with regard to the guiding members to further flex the skin around the guiding members and needle.

According to the invention each guiding member comprises a curved bar, wherein the apex of the curvature is provided for engagement with the skin during introduction of the needle parallel to the skin. The bar is curved such that its free extremity extends obliquely away from the axis of the needle beyond the tip of the needle. In this way it is possible to start introduction of the needle obliquely with regard to the skin for piercing of this skin. After that following the bar turns the apparatus downwardly to the skin of the animal through the curved part of the bar such that the needle can be further introduced through a smooth introduction movement where it is guaranteed that the needle will always be parallel to the skin of the animal, whatever curve the skin surface follows.

The needle may be displaceable with regard to the guiding members. This can be realized in several ways but one of them is to prevent the needle extending from the housing in the condition of non use. According to a further embodiment of the invention the needle is a hollow needle and means are provided to transfer products therethrough.

Transport of such products can more particularly be effected when the transfer means and guiding means are interconnected. This means that after introduction of the needle in the desired position below the skin of the animal through operation of the apparatus the guiding means and the transfer means remain stationary whilst the needle is retracted. The product cannot move along with the needle because of the presence of the transfer means and will be placed on the desired location.

For ease of introduction the tip of the needle is preferably bevelled. This is realized such that the farthest extremity of such a tip will be remote from the skin during introduction movement under the skin after the needle has been introduced..

The invention will be further elucidated referring to a preferred embodiment of the invention shown in the drawing, wherein:
Fig. 1 shows (partially in cross section) an apparatus according to the invention;
Fig. 2 shows in elevation the front part of the apparatus according to Fig. 1;
Fig. 3 shows in plan view the front part of the apparatus according to Fig. 1;
Fig. 4 shows the use of the apparatus according to Fig. 1 during introduction in the head of an animal and
fig. 5 a cross section according to line V-V in Fig. 4.

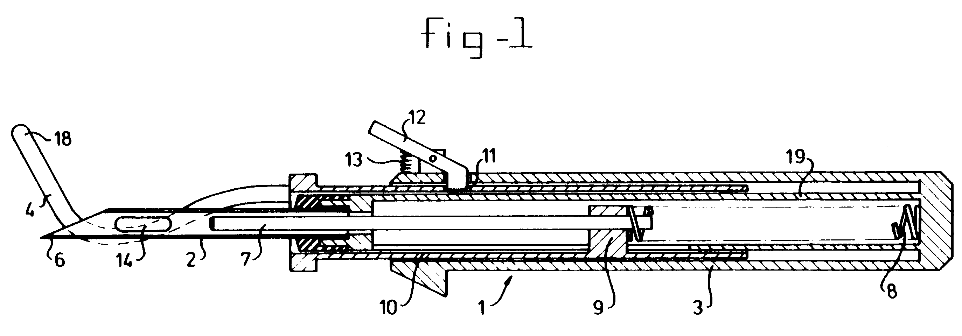

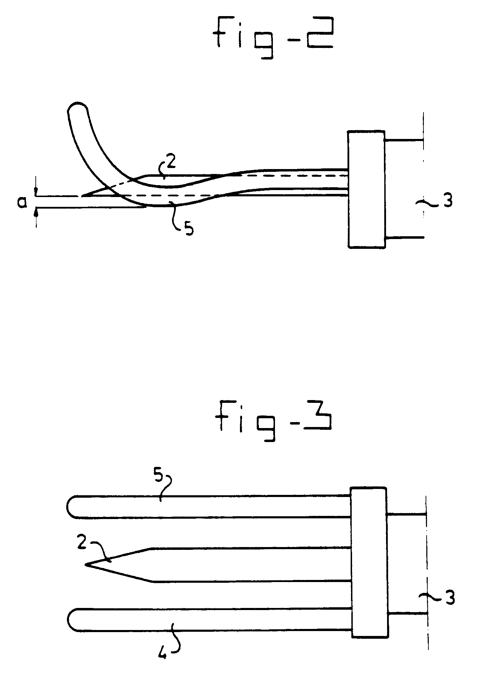

In Fig. 1 an injector 1 is shown comprising a housing 3 and needle 2. The needle 2 is rigidly connected to housing 3 through sleeve 19. Between sleeve 19 and outer part of housing 3 sleeve 10 is provided to which guiding members 4 and 5 (see also fig.3) are connected. The outer part of housing 3 is provided with a trigger 12 which can be actuated against a spring 13. Trigger 12 is designed to engage an opening 11 in sleeve 10. Sleeve 10 is provided with a support block 9 in which a pushing rod 7 is mounted. One side of pushing rod 7 is introduced in hollow needle 2 whilst a protruding end of it acts to receive a compression spring 8 which on the other hand engages on housing 3. Guiding member 4 extends from its attachment point to sleeve 10 first substantially parallel to the needle, then curves downwardly below the needle and after that upwardly well above the needle to end in extremity 18. Extremity 18 is somewhat beyond bevelled tip 6 of the needle. 14 indicates a product to be introduced in an animal such as a drug or a transponder used for identifying animals.

The apparatus shown in fig. 1 functions as follows.

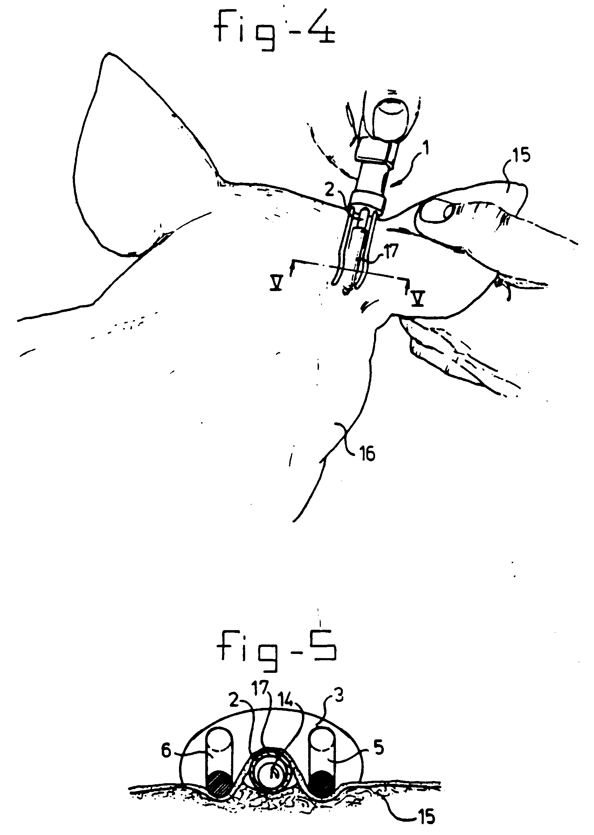

In the position shown in fig. 1 spring 8 is under compression. In this condition shown the needle is introduced under the skin of the animal as will be further elucidated with regard to fig. 4 and 5. After complete introduction of the needle and still in the position of fig. 1 trigger 12 is actuated. Because of force of spring 8 acting on support block 9 and releasing of sleeve 10 through trigger 12 sleeve 10 moves outwardly relative to housing 3. During this movement both guiding members 4 and 5 and pushing rod 7 will move outwardly. However, in practice this means that guiding members 4 and 5 and transfer rod 7 will remain stationary whilst needle 2 and housing 3 will retract from the animal. In the end position not shown the needle will be completely received in sleeve 10 so that there is no danger from the tip 6 of the needle. During this retracting movement product 14 will remain in position under the skin of the animal and will be placed after removing apparatus 1 from the animal.

In fig. 4 and 5 the introduction of the injector 1 according to the invention is shown. To be able to pierce the skin of the animal injector 1 is introduced obliquely to the skin of the animal. This is facilitated by the shape of extremity 18 of guiding members 4 and 5. After piercing the skin through bevelled part 6 of the needle the injector is tilted downwardly to the skin of the animal. This tilting movement is guided by the curved shape of guiding members 4 and 5. After that the injector is displaced parallel to the skin of the animal. The skin is folded between the guiding members 4 and 5 pushing the skin downwardly in fig. 4 and the needle 2 trying to move the skin upwardly. This is shown more clearly in fig. 5. In this way the skin is also released from tissue below the skin and bone, cartilage or tissue being below the skin.

The distance between the contact surface of the curved guiding members 4 and 5 and the upper surface of the needle engaging to the lower (inner) part of the skin can be varied according to the use of the needle. In fig. 2 an embodiment is shown wherein the needle is relatively "high" with regard to the contact face of the guided member. For example distance "a" can be several millimetres. However, as indicated above it is also possible that the contact surface of the guiding member is somewhat higher.

Although the invention is described above relating to a preferred embodiment of the invention it is possible to realize all kinds of changes without leaving the scope of the appended claims.

## Claims

1. Apparatus (1) for subcutaneous injection of a needle (2) in a living being, substantially tangentially to the skin of said living being, said apparatus comprising a housing (3), a needle (2) extending from said housing and two guiding means (4,5) having a portion substantially parallel to the needle and a curved end portion for determining the position of the needle relative to the living being extending substantially in the same longitudinal direction as the needle and arranged in use to outwardly engage the skin with a lower face, wherein the skin is positioned between the two guiding members, the guiding members and needle being positioned such relative to each other that in the position of introduction of the needle the lower face of each guiding member near the tip of the needle is at least at the same level of or lower than the lower part of the tip of the needle, characterised in that each guiding member (4,5) comprises a bar or rod having a free end (18) and in that the free ends (18) of the curved bars or rods extend obliquely away from the axis of the needle (2), the tip (6) of the needle (2) extending along the direction of the axis beyond the distal curved portion of the free ends of the bars or rods.

2. Apparatus according to claim 1, wherein the needle is a hollow needle and means (7) are provided to transfer products (14) through said needle.

3. Apparatus according to claim one of the preceding claims, wherein the guiding members and needle are positioned such relative to each other that in the position of use the face of each guiding member which in use is in contact with the skin is at least at the same level of the part of the needle which in use is remote from the skin.

4. Apparatus according to one of the preceding claims, wherein the guiding member comprises a curved bar, the apex of the curvature being provided for engagement with the skin during introduction of the needle to maintain it parallel to the skin.

5. Apparatus according to one of the previous claims, wherein the needle is displaceable with regard to the guiding members.

6. Apparatus according to claim 6 or 7, wherein the transfer means and guiding means are interconnected.

7. Apparatus according to one of the previous claims, wherein the tip of the needle is bevelled, in such a way that the farthest extremity of the tip is positioned remote from the skin of the living being after introduction of the needle under the skin.

## Patentansprüche

1. Vorrichtung (1) zur subkutanen Injektion einer Nadel (2) in ein Lebewesen im wesentlichen tangential zu der Haut des Lebewesens, wobei die Vorrichtung ein Gehäuse (3), eine sich von dem Gehäuse erstreckende Nadel (2) sowie zwei Führungsmittel (4, 5) aufweist, die einen im wesentlichen zu der Nadel parallelen Abschnitt und einen gekrümmten Endabschnitt zur Bestimmung der Position der Nadel relativ zu dem Lebewesen aufweisen, der sich im wesentlichen in der gleichen Längsrichtung wie die Nadel erstreckt und bei Verwendung derart angeordnet ist, daß er mit einer Unterseite an der Haut angreift, wobei die Haut zwischen den zwei Führungselementen positioniert ist, wobei die Führungselemente und die Nadel derart zueinander positioniert sind, daß in der Einführungsposition der Nadel die Unterseite jedes Führungselements nahe der Spitze der Nadel wenigstens auf der gleichen Höhe oder unter dem unteren Teil der Nadelspitze liegt, dadurch gekennzeichnet, daß jedes Führungselement (4, 5) einen Stab oder einen Stift mit einem freien Ende (18) aufweist, und daß sich die freien Enden (18) der gekrümmten Stäbe oder Stifte schräg von der Achse der Nadel (2) wegerstrecken, wobei sich die Spitze (6) der Nadel (2) längs der Richtung der Achse über den distalen gekrümmten Abschnitt der freien Enden der Stäbe oder Stifte hinauserstreckt.

2. Vorrichtung nach Anspruch 1, bei welcher die Nadel eine Hohlnadel ist und Mittel (7) vorgesehen sind, um durch die Nadel Produkte (14) zu übertragen.

3. Vorrichtung nach einem der vorhergehenden Ansprüche, bei welcher die Führungselemente und die Nadel derart zueinander positioniert sind, daß in der Verwendungsposition die Seite jedes Führungselements, die bei Verwendung mit der Haut in Kontakt steht, wenigstens auf der gleichen Höhe wie der Teil der Nadel liegt, der bei Verwendung von der Haut entfernt ist.

4. Vorrichtung nach einem der vorhergehenden Ansprüche, bei welcher das Führungselement einen gekrümmten Stab aufweist, wobei der Scheitelpunkt der Krümmung während des Einführens der Nadel zum Eingriff mit der Haut vorgesehen ist, um sie parallel zu der Haut zu halten.

5. Vorrichtung nach einem der vorhergehenden Ansprüche, bei welcher die Nadel bezüglich der Führungselemente verschiebbar ist.

6. Vorrichtung nach Anspruch 6 oder 7, bei welcher die Übertragungsmittel und die Führungsmittel miteinander verbunden sind.

7. Vorrichtung nach einem der vorhergehenden Ansprüche, bei welcher die Spitze der Nadel derart abgeschrägt ist, daß das weiteste Ende der Spitze nach dem Einführen der Nadel unter die Haut fern von der Haut des Lebewesens positioniert ist.

## Revendications

1. Dispositif (1) pour l'introduction sous-cutanée d'une aiguille (2) dans un être vivant, sensiblement tangentiellement à la peau dudit être vivant, ledit dispositif comprenant un boitier (3), une aiguille (2) s'étendant à partir dudit boitier et deux moyens de guidage (4, 5) possédant une partie sensiblement parallèle à l'aiguille et une partie d'extrémité courbe pour déterminer la position de l'aiguille par rapport à l'être vivant et qui s'étend sensiblement dans la même direction longitudinale que l'aiguille et est disposée de manière à être utilisée pour venir en contact extérieurement, par une face inférieure, avec la peau, dans lequel la peau est disposée entre les deux éléments de guidage, les éléments de guidage et l'aiguille étant positionnés réciproquement de telle sorte que, dans la position d'introduction de l'aiguille, la face inférieure de chaque élément de guidage à proximité de la pointe de l'aiguille est au moins au même niveau ou à un niveau plus bas que la partie inférieure de la pointe de l'aiguille, caractérisé en ce que chaque élément de guidage (4, 5) comprend une barre ou une tige possédant une extrémité libre (18) et en ce que les extrémités libres (18) des barres ou tiges courbes s'étendent obliquement à partir de l'axe de l'aiguille (2), la pointe (6) de l'aiguille (2) s'étendant dans la direction de l'axe au-delà de la partie distale coudée des extrémités libres des barres ou des tiges.

2. Dispositif selon la revendication 1, dans lequel l'aiguille est une aiguille creuse et des moyens (7) sont prévus pour transférer des produits (14) au travers de ladite aiguille.

3. Dispositif selon l'une des revendications précédentes, dans lequel les moyens de guidage de l'aiguille sont positionnés relativement entre eux de telle sorte que, dans la position d'utilisation, la face de chaque élément de guidage qui, en cours d'utilisation, est en contact avec la peau, est au moins située au même niveau que la partie de l'aiguille qui, en cours d'utilisation, est éloignée de la peau.

4. Dispositif selon l'une des revendications précédentes, dans lequel l'élément de guidage comprend une barre courbe, le sommet de la courbe étant prévu de manière à s'appliquer contre la peau pendant l'introduction de l'aiguille afin de la maintenir parallèle à la peau.

5. Dispositif selon l'une des revendications précédentes, dans lequel l'aiguille est déplaçable par rapport aux éléments de guidage.

6. Dispositif selon la revendication 5, dans lequel les moyens de transfert et les moyens de guidage sont interconnectés.

7. Dispositif selon l'une des revendications précédentes, dans lequel la pointe de l'aiguille est biseautée de telle sorte que l'extrémité la plus éloignée de la pointe est positionnée à distance de la peau de l'être vivant après introduction de l'aiguille sous de la peau.
